# EUROPÄISCHE PATENTSCHRIFT

(11) **EP 1 261 591 B1**
(45) Veröffentlichungstag und Bekanntmachung des Hinweises auf die Patenterteilung: **08.03.2006**
(21) Anmeldenummer: 01274021.3
(22) Anmeldetag: 06.11.2001
(51) Int. Cl.: C07D 271/12, C07D 413/04, C07D 417/04, C07D 285/14, A61Q 5/10

(54) **7-NITRO-2,1,3-BENZOXADIAZOL-UND 7-NITRO-2,1,3-BENZTHIADIAZOL DERIVATE SOWIE DIESE VERBINDUNGEN ENTHALTENDE FÄRBEMITTEL FÜR KERATINFASERN**
7-NITRO-2,1,3-BENZOXADIAZOLE DERIVATIVES AND 7-NITRO-2,1,3-BENZTHIADIAZOLE DERIVATIVES AND DYES, WHICH CONTAIN THESE COMPOUNDS AND WHICH ARE USED FOR DYING KERATIN FIBERS
DERIVES DE 7-NITRO-2,1,3-BENZOXADIAZOL-ET 7-NITRO-2,1,3-BENZTHIADIAZOL ET COLORANTS POUR FIBRES DE KERATINE CONTENANT LESDITS COMPOSES

(30) Priorität: 21.03.2001 DE 10113699
(43) Veröffentlichungstag der Anmeldung: 04.12.2002
(73) Patentinhaber: Wella Aktiengesellschaft, 64274 Darmstadt (DE)
(72) Erfinder: UMBRICHT, Gisela, CH-1723 Marly (CH); BRAUN, Hans-Jürgen, CH-3182 Überstorf (CH); OBERSON, Sylviane, CH-1730 Ecuvillens (CH); MÜLLER, Catherine, CH-1723 Marly (CH)
(86) Internationale Anmeldenummer: PCT/EP2001/012806
(87) Internationale Veröffentlichungsnummer: WO 2002/076961

(56) Entgegenhaltungen:
- EP-A- 0 861 835
- US-A- 5 705 649
- KIND, J. ET AL: "Preparation of 4-(2-aryl-2-oxo-ethylidene)-5,7-dinitroben zofurazan derivatives" J. PRAKT. CHEM./CHEM.-ZTG. (1994), 336(5), 439-43 , XP001057756
- HALLE ET AL: "Nucleophilic Displacement of Hydrogen in 4,6-Dinitro-Benzofuran and -Benzofurazan Synthesis of Some Novel 7-Substituted 4,6-Dinitro-Benzofurans and -Benzofurazans" TETRAHEDRON LETTERS, Bd. 26, Nr. 10, 1985, Seiten 1307-1210, XP001057882
- PESIN ET AL: "Synthesis and Properties of 4-and 5-(Bromomethyl)-2,1,3-Benzothiazoles" JOURNAL OF GENERAL CHEMISTRY OF THE USSR(ENGLISH TRANSLATION), Bd. 34, 1964, Seiten 1270-1273, XP001057853

## Beschreibung

Gegenstand der vorliegenden Erfindung sind neue 7-Nitro-2,1,3-benzoxadiazol- oder 7-Nitro-2,1,3-benzthiadiazol-Derivate und diese Verbindungen enthaltende Färbemittel für Keratinfasern, insbesondere menschliche Haare.

Für die farbverändernde Behandlung keratinhaltiger Fasern, zum Beispiel menschlicher Haare, Wolle oder Pelzen, werden in der Regel zwei Färbeverfahren angewendet. Im ersten Verfahren wird die Färbung mit sogenannten oxidativen oder permanenten Färbemitteln unter Verwendung einer Mischung aus verschiedenen Entwicklersubstanzen und Kupplersubstanzen und eines Oxidationsmittels erzeugt. Bei Bedarf können zur Abrundung des Färbeergebnisses oder zur Erzeugung von besonderen Farbeffekten sogenannte direktziehende (nicht-oxidative) Farbstoffe zugesetzt werden. Das zweite Verfahren bedient sich ausschliesslich direktziehender Farbstoffe, die in einer geeigneten Trägermasse auf die Fasern aufgebracht werden. Dieses Verfahren ist einfach anzuwenden, ausgesprochen mild und zeichnet sich durch eine geringe Schädigung der Keratinfaser aus. An die hierbei verwendeten direktziehenden Farbstoffe werden eine Vielzahl von Anforderungen gestellt. So müssen sie in toxikologischer und dermatologischer Hinsicht unbedenklich sein und die Erzielung von Färbungen in der gewünschten Intensität ermöglichen, was unter anderem auch eine ausreichende Wasserlöslichkeit voraussetzt. Ausserdem wird für die erzielten Färbungen eine gute Lichtechtheit, Säureechtheit und Reibechtheit sowie eine gute Stabilität im Waschverhalten gefordert.

EP 0 861 835 beschreibt oxidative Färbemittel für Keratin fasern, US 5 705 649 beschreibt Färbemittel, die im Bereich der Elektrophorese angewandt werden

Für ein direktziehendes (nicht-oxidatives) Färbemittel für Keratinfasern wird in der Regel eine Kombination von verschiedenen nicht-oxidativen Farbstoffen benötigt. Da die Auswahl an in Färbemitteln für Keratinfasern einsetzbaren roten und blauen Farbstoffen beschränkt ist, besteht weiterhin ein Bedarf an derartigen Farbstoffen.

Gegenstand der vorliegenden Erfindung sind neue 7-Nitro-2,1,3-benzoxadiazol-Derivate und 7-Nitro-2,1,3-benzthiadiazol-Derivate der allgemeinen Formel (I) 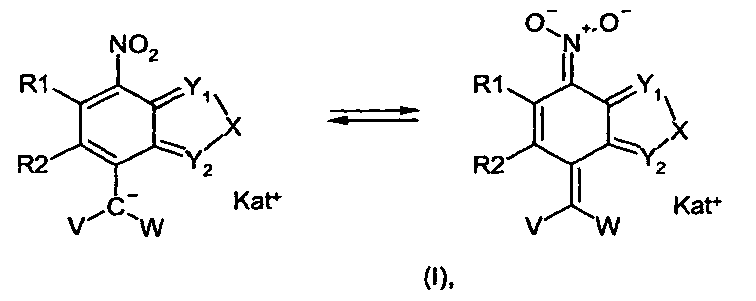
wobei gilt:
**X** ist gleich Sauerstoff oder Schwefel;
**Y1** und **Y2** können gleich oder verschieden sein und stehen unabhängig voneinander für ein Stickstoffatom oder eine Stickstoffmonoxid-Gruppe (NO);
**R1** und **R2** können gleich oder verschieden sein und stellen unabhängig voneinander Wasserstoff, ein Halogenatom (F, Cl, Br, I), eine (C₁-C₄)-Alkylgruppe, eine mit einem Halogenatom substituierte (C₁-C₄)-Alkylgruppe, eine (C₁-C₄)-Alkoxygruppe, eine Nitrogruppe oder eine NR^{a}R^{b-}Gruppe dar, wobei die Reste R^{a} und R^{b} gleich oder verschieden sein können und unabhängig voneinander Wasserstoff, eine (C₁-C₄)-Alkylgruppe, einen gegebenfalls substituierten aromatischen Carbozyklus oder eine (C₁-C₄)-Alkancarbonylgruppe darstellen, oder R^{a} und R^{b} gemeinsam mit dem Stickstoffatom einen heterozyklischen (C₃-C₆)- Rest (beispielsweise eine Imidazolidino-, Piperidino-, Pyrrolidino-, Pyrazolidino-, Piperazino- oder Morpholino-Gruppe) bilden;
**V** ist gleich Wasserstoff, einem aliphatischen Rest, einem aromatischen isozyklischen Rest, einem aromatischen heterozyklischen Rest, einer Cyanogruppe oder einer Carbonylfunktion (CO)-R3, wobei R3 für Wasserstoff, eine Hydroxygruppe, eine (C₁-C₄)-Alkoxygruppe, eine Aminogruppe, eine (C₁-C₄)-Alkylaminogruppe, eine (C₁-C₆)-Alkylgruppe oder eine Arylgruppe steht;
**W** stellt eine Cyanogruppe oder eine Carbonylfunktion (CO)-R4 dar, wobei R4 für Wasserstoff, eine Hydroxygruppe, eine (C₁-C₄)-Alkoxygruppe, eine Aminogruppe, eine (C₁-C₄)-Alkylaminogruppe, eine (C₁-C₆)-Alkylgruppe oder eine Arylgruppe steht; alternativ können **V** und **W** auch gemeinsam ein aliphatisches oder aromatisches isozyklisches oder heterozyklisches Ringsystem bilden; und
**Kat**^{**+**} entspricht einem Alkalikation, einem Erdalkalikation, einer quaternären Ammoniumgruppe, einer quaternären Phosphoniumgruppe oder einer Sulfoniumgruppe;

unter der Bedingung, dass **W** nicht gleich einer CO-Phenylgruppe, einer CO-(4-Bromphenyl-)-Gruppe, einer CO-(4-Chlorphenyl-)-Gruppe, einer CO-(4-Methylphenyl-)-Gruppe oder einer CO-(4-Methoxyphenyl-)Gruppe ist, wenn **R1** gleich Wasserstoff ist, **V** gleich Wasserstoff oder einem unsubstituierten Pyridylrest ist, **R2** gleich einer Nitrogruppe ist, **X** gleich Sauerstoff ist und **Y1** und **Y2** gleich einem Stickstoffatom sind.

Eingeschlossen sind auch alle weiteren tautomeren Formen der allgemeinen Formel (I).

Bevorzugt sind Mittel mit Verbindungen der Formel (I) in denen gilt:
**X** ist gleich Sauerstoff oder Schwefel ;
**Y1** und **Y2** können gleich oder verschieden sein und stehen unabhängig voneinander für ein Stickstoffatom oder eine Stickstoffmonoxid-Gruppe (NO); **R1** und **R2** können gleich oder verschieden sein und stellen unabhängig voneinander Wasserstoff, ein Halogenatom (F, Cl, Br, I), eine (C1-C4)-Alkylgruppe oder eine Nitrogruppe dar;
**V** ist gleich Wasserstoff, einem aliphatischen Rest, einem aromatischen isozyklischen Rest, einem aromatischen heterozyklischen Rest, einer Cyanogruppe oder einer Carbonylfunktion (CO)-R3, wobei R3 für Wasserstoff, eine Hydroxygruppe, eine (C₁-C₄)-Alkoxygruppe, eine Aminogruppe, eine (C₁-C₄)-Alkylaminogruppe, eine (C₁-C₆)-Alkylgruppe oder eine Arylgruppe steht;
**W** stellt eine Cyanogruppe oder eine Carbonylfunktion (CO)-R4 dar, wobei R4 für Wasserstoff, eine Hydroxygruppe, eine (C₁-C₄)-Alkoxygruppe, eine Aminogruppe, eine (C₁-C₄)-Alkylaminogruppe, eine (C₁-C₆)-Alkylgruppe oder eine Arylgruppe steht; alternativ können **V** und **W** auch gemeinsam ein aliphatisches oder aromatisches isozyklisches oder heterozyklisches Ringsystem bilden; und
**Kat**^{**+**} entspricht einem Alkalikation, einem Erdalkalikation, einer quaternären Ammoniumgruppe, einer quaternären Phosphoniumgruppe oder einer Sulfoniumgruppe.

Besonders bevorzugte 7-Nitro-2,1,3-benzoxadiazol-Derivate der Formel (I) sind das 4-(Dicyanomethyl)-7-nitro-2,1,3-benzoxadiazol-natriumsalz, 4-(1-Cyano-2-ethoxy-2-oxoethyl)-7-nitro-2,1,3-benzoxadiazol-natriumsalz, 4-(Dicyanomethyl)-7-nitro-2,1,3-benzoxadiazol-N-oxid natriumsalz, 4-(Dihydro-2,4,6(1H,5H)-pyrimidintrion-5-yl)-7-nitro-2,1,3-benzoxadiazol-natriumsalz, 4-(1-Cyano-3.3-dimethyl-2-oxobutyl)-7-nitro-2,1,3-benzoxadiazol-natriumsalz, 4-(Bis(methoxycarbonyl)-7-nitro-2,1,3-benzoxadiazol-natriumsalz, 4-(4,5-Dihydro-3-methyl-1-phenyl-1H-pyrazol-5-on-4-yl)-7-nitro-2,1,3-benzoxadiazol-natriumsalz, 4-(Cyano-(4-nitrophenyl)-methyl)-7-nitro-2,1,3-benzoxadiazol-natriumsalz, 4-((Aminocarbonyl)-cyano-methyl)-7-nitro-2,1,3-benzoxa-diazol-1-oxid-natriumsalz, 4-(1-Cyano-2-ethoxy-2-oxo-ethyl)-7-nitro-2,1,3-benzoxa-diazol-1-oxidnatriumsalz, 4-(1,3-Cyclohexandion-2-yl)-7-nitro-2,1,3-benzoxadiazol-natriumsalz, 4-(Carboxy-cyanomethyl)-7-nitro-2,1,3-benzoxadiazol-natriumsalz, 4-(2-Ethoxy-1-nitro-2-oxoethyl)-7-nitro-2,1,3-benzoxadiazol-natriumsalz, 4-((Aminocarbonyl)cyanomethyl)-7-nitro-2,1,3-benzoxadiazol-natriumsalz, 4-(Dihydro-2-thioxo-4,6(1H,5H)-pyrimidindion-5-yl)-7-nitro-2,1,3-benzoxadiazol-1-oxid-natriumsalz, 4-(1,3-Dioxo-indan-2-yl)-7-nitro-2,1,3-benzoxadiazol-natriumsalz, 4-(2-Oxo-2,3-dihydro-1H-indol-3-yl)-7-nitro-2,1,3-benzoxadiazol-natriumsalz, 4-(4-Oxo-2-thioxo-thiazolidin-5-yl)-7-nitro-2,1,3-benzoxadiazol-natriumsalz, 4-(Dihydro-6-thioxo-2,4-(1H,5H)-pyrimidin-dion-3-yl)-7-nitro-2,1,3-benzoxadiazol-natriumsalz, 4-(1-Cyano-2-oxo-2-phenylethyl)-2,1,3-benzoxadiazol-natriumsalz und 4-(Cyano-(2-nitrophenyl)-methyl)-7-nitro-2,1,3-benzoxadiazol-natriumsalz.

Besonders bevorzugte 7-Nitro-2,1,3-benzthiadiazol-Derivate der Formel (I) sind das 4-(Dicyanomethyl)-7-nitro-2,1,3-benzthiadiazol-natriumsalz, 4-(1-Cyano-2-ethoxy-2-oxoethyl)-7-nitro-2,1,3-benzothiadiazolnatriumsalz, 4-(Cyano-(4-nitrophenyl)-methyl)-7-nitro-2,1,3-benzthiadiazol-natriumsalz, 4-(Dicyanomethyl)-7-nitro-2,1,3-benzthiadiazol-N-oxidnatriumsalz, 4-(Dihydro-2,4,6(1H,5H)-pyrimidintrion-5-yl)-7-nitro-2,1,3-benzthiadiazol-natriumsalz, 4-(1-Cyano-3,3-dimethyl-2-oxobutyl)-7-nitro-2,1,3-benzthiadiazol-natriumsalz, 4-(Bis(methoxycarbonyl)-7-nitro-2,1,3-benzthiadiazol-natriumsalz, 4-(4,5-Dihydro-3-methyl-1-phenyl-1H-pyrazol-5-on-4-yl)-7-nitro-2,1,3-benzthiadiazol-natriumsalz, 4-(1,3-Cyclohexandion-2-yl)-7-nitro-2,1,3-benzthiadiazol-natriumsalz. 4-(Carboxy-cyanomethyl)-7-nitro-2,1,3-benzthiadiazol-natriumsalz, 4-(2-Ethoxy-1-nitro-2-oxoethyl)-7-nitro-2,1,3-benzthiadiazol-natriumsalz, 4-((Aminocarbonyl)cyanomethyl)-7-nitro-2,1,3-benzthiadiazol-natriumsatz, 4-(Dihydro-2-thioxo-4,6(1H,5H)-pyrimidindion-5-yl)-7-nitro-2,1,3-benzthiadiazol-1-oxid-natriumsalz, 4-(1,3-Dioxo-indan-2-yl)-7-nitro-2,1,3-benzthiadiazol-natriumsalz, 4-(2-Oxo-2,3-dihydro-1H-indol-3-yl)-7-nitro-2,1,3-benzthiadiazol-natriumsalz, 4-(4-Oxo-2-thioxo-thiazolidin-5-yl)-7-nitro-2,1,3-benzthiadiazol-natriumsalz, 4-(Dihydro-6-thioxo-2,4,(1H,5H)-pyrimidin-dion-3-yl)-7-nitro-2,1,3-benzthiadiazol-natriumsalz, 4-(1-Cyano-2-oxo-2-phenylethyl)-2,1,3-benzthiadiazol-natriumsalz und 4-(Cyano-(2-nitrophenyl)-methyl)-7-nitro-2,1,3-benzoxadiazoi-natriumsatz.

Die Verbindungen der Formel (I) können in Analogie zu den Ausführungsbeispielen auf einfache Weise durch eine einstufige Reaktion von Nitrosubstituierten Benzofurazanen oder deren Thiaanaloga mit CH-aktiven Verbindungen nach dem nachfolgenden Schema 1 hergestellt werden, 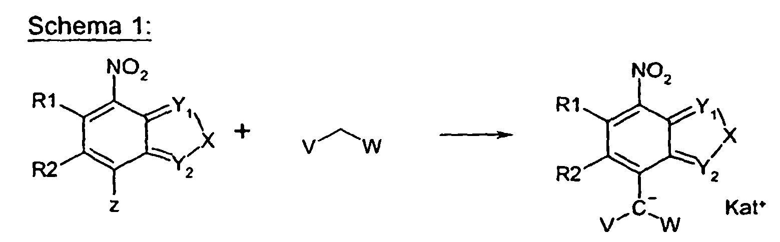
wobei R1, R2, X, Y1, Y2, V und W die für die Formel (I) vorstehend beschriebene Bedeutung haben und Z ein Wasserstoffatom, ein Halogenatom (F, Cl, Br, I) oder eine Alkoxygruppe (Methoxy, Ethoxy) darstellt.

Die neuen Farbstoffderivate der Formel (I) besitzen nicht nur eine gute Wasserlöslichkeit, vielmehr zeichnen sich diese neuen Farbstoffderivate ebenfalls durch ein gleichmässiges Aufziehverhalten und eine grosse Waschstabilität aus. Die erfindungsgemässen Farbstoffe ermöglichen eine Färbung von Keratinfasern, insbesondere von menschlichen Haaren, aber auch von Wolle oder Pelzen, unter schonenden und hautverträglichen Bedingungen.

Ein weiterer Gegenstand der vorliegenden Erfindung ist daher die Verwendung der Verbindungen der Formel (I) als Farbstoff in Färbemitteln für Keratinfasern, insbesondere in Haarfärbemittein.

Ein weiterer Gegenstand der vorliegenden Erfindung ist ein Mittel zum Färben von Keratinfasern, insbesondere menschlicher Haare, welches in einer geeigneten Kosmetikgrundlage mindestens eine Verbindung der allgemeinen Formel (I) enthält.

Die Einsatzmenge der Verbindungen der Formel (I) beträgt in dem erfindungsgemässen Färbemittel vorzugsweise etwa 0,01 bis 10 Gewichtsprozent, wobei eine Einsatzmenge von etwa 0,1 bis 8 Gewichtsprozent besonders bevorzugt ist.

Das Färbemittel kann, insbesondere wenn es ein Haarfärbemittel ist, in Form einer wässrigen oder wässrig-alkoholischen Lösung, einer Creme, eines Gels, einer Emulsion oder eines Aerosolschaumes vorliegen, wobei das Haarfärbemittel sowohl in Form eines Einkomponentenpräparates als auch in Form eines Mehrkomponentenpräparates, beispielsweise in Form eines Zweikomponentenpräparates, bei dem das Farbstoffderivat der Formel (I) getrennt von den übrigen Bestandteilen abgepackt wird und die Herstellung des gebrauchsfertigen Haarfärbemittels erst unmittelbar vor der Anwendung durch Vermischen der beiden Komponenten erfolgt, konfektioniert sein kann.

Das Färbemittel kann neben Wasser auch organische Lösungsmittel, beispielsweise aliphatische oder aromatische Alkohole, wie zum Beispiel Ethanol, Isopropanol, 1,2-Propandiol, 1-Methoxypropan-2-ol, 1-Ethoxypropan-2-ol, Diethylenglykolmonomethylether oder Diethylenglykolmonoethylether, Dipropylenglykolmonomethylether, Dipropylenglykolmonoethylether, Benzylalkohol, Benzyloxyethanol, Phenylethylalkohol, Phenoxyethanol, Zimtalkohol und Glykolether, insbesondere Ethanol, Isopropanol oder Benzylalkohol, enthalten, wobei der Wassergehalt in der Regel etwa 25 bis 95 Gewichtsprozent, vorzugsweise etwa 30 bis 85 Gewichtsprozent, beträgt, während der Gehalt an dem organischen Lösungsmittel oder Lösungsmittelgemisch bei etwa 5 bis 30 Gewichtsprozent liegt.

Das Färbemittel kann weiterhin alle für derartige Zubereitungen bekannten und üblichen Zusatzstoffe, beispielsweise Parfümöle, Komplexbildner, Wachse, Konservierungsstoffe, Verdicker, Alginate, Guar Gum, haarpflegende Substanzen, wie zum Beispiel Lanolinderivate, oder anionische, nichtionische oder amphotere oberflächenaktive Substanzen enthalten. Vorzugsweise werden amphotere oder nicht-ionische oberflächenaktive Substanzen, beispielsweise Betaintenside, Propionate und Glycinate, wie zum Beispiel Cocoampho-glycinate oder Cocoamphodiglycinate, ethoxylierte Tenside mit 1 bis 1000 Ethylenoxid-Einheiten, vorzugsweise mit 1 bis 300 Ethylenoxid-Einheiten, wie zum Beispiel Glyceridalkoxylate, beispielsweise mit 25 Ethylenoxid-Einheiten ethoxyliertes Rizinusöl, Polyglykolamide, ethoxylierte Alkohole und ethoxylierte Fettalkohole (Fettalkoholalkoxylate) und ethoxylierte Fettsäurezuckerester, insbesondere ethoxylierte Sorbitanfettsäureester, eingesetzt. Die vorgenannten Bestandteile werden in den für solche Zwecke üblichen Mengen verwendet, zum Beispiel die oberflächenaktiven Substanzen in einer Konzentration von 0,1 bis 30 Gewichtsprozent, und die Pflegestoffe in einer Menge von 0,1 bis 5 Gewichtsprozent.

Das Färbemittel kann je nach gewünschtem Farbton neben den Farbstoffen der Formel (I) gegebenenfalls zusätzlich noch weitere bekannte direktfärbende Farbstoffe aus der Gruppe der anionischen, kationischen, nicht-ionischen oder amphoteren Farbstoffe, Nitrofarbstoffe, Azofarbstoffe, Anthrachinonfarbstoffe und Dispersionsfarbstoffe enthalten, wobei diese Farbstoffe einzeln oder im Gemisch miteinander eingesetzt werden können.

Die vorstehend genannten zusätzlichen direktziehenden Farbstoffe können in einer Gesamtmenge von etwa 0,01 bis 4 Gewichtsprozent enthalten sein, wobei der Gesamtgehalt an Farbstoffen in dem erfindungsgemässen Färbemittel vorzugsweise etwa 0,01 bis 10 Gewichtsprozent, insbesondere 0,1 bis 5 Gewichtsprozent, beträgt.

Das Färbemittel weist einen pH-Wert von etwa 3 bis 10, vorzugsweise etwa 4 bis 10, auf. Zur Einstellung des pH-Wertes werden je nach gewünschtem pH-Wert entweder organische oder anorganische Säuren oder aber organische oder anorganische Basen verwendet.

Als geeignete Säuren sind insbesondere die folgenden Säuren zu nennen: α-Hydroxycarbonsäuren, wie zum Beispiel Glykolsäure, Milchsäure, Weinsäure, Zitronensäure, Äpfelsäure, Ascorbinsäure, Gluconsäurelacton, Essigsäure, Salzsäure oder Phosphorsäure, sowie Mischungen dieser Säuren.

Als geeignete Basen sind insbesonders Natriumcarbonat, Natriumhydrogencarbonat, Kaliumcarbonat, Kaliumhydrogencarbonat, Natriumphosphat, Borax (Na₂B₄O₇ x 10H₂O), Dinatriumhydrogenphosphat, Alkanolamine, beispielsweise Monoethanolamin oder Triethanolamin, Ammoniak, Aminomethylpropanol und Natriumhydroxid, sowie Mischungen dieser Basen, zu nennen.

Das vorstehend beschriebene Färbemittel kann weiterhin für kosmetische Mittel übliche natürliche oder synthetische Polymere beziehungsweise modifizierte Polymere natürlichen Ursprungs enthalten, wodurch gleichzeitig mit der Färbung eine Festigung der Haare erreicht wird. Solche Mittel werden im allgemeinen als Tönungsfestiger oder Farbfestiger bezeichnet.

Von den für diesen Zweck in der Kosmetik bekannten synthetischen Polymeren seien beispielsweise Polyvinylpyrrolidon, Polyvinylacetat, Polyvinylalkohol oder Polyacrylverbindungen wie Polyacrylsäure oder Polymethacrylsäure, basische Polymerisate von Estern der Polyacrylsäure, Polymethylacrylsäure und Aminoalkoholen, beispielsweise deren Salze oder Quaternisierungsprodukte, Polyacrylnitril, Polyvinylacetate sowie Copolymerisate aus derartigen Verbindungen, wie zum Beispiel Polyvinylpyrrolidon-Vinylacetat, erwähnt; während als natürliche Polymere oder modifizierte natürliche Polymere beispielsweise Chitosan (deacetyliertes Chitin) oder Chitosanderivate, eingesetzt werden können.

Die vorgenannten Polymere können in dem Färbemittel in den für solche Mittel üblichen Mengen, insbesondere in einer Menge von etwa 1 bis 5 Gewichtsprozent, enthalten sein. Der pH-Wert des erfindungsgemässen Tönungsfestigers oder Farbfestigers beträgt vorzugsweise etwa 6 bis 9.

Die Anwendung des Färbemittels erfolgt bei der Färbung von Haaren in der Regel indem man eine für die Haarfärbung ausreichende Menge, je nach Haarlänge etwa 30 bis 120 Gramm, des Haarfärbemittels auf das Haar aufträgt, das Haarfärbemittel bei etwa 15 bis 45 Grad Celsius etwa 1 bis 60 Minuten, vorzugsweise 5 bis 30 Minuten, einwirken läßt, das Haar anschliessend gründlich mit Wasser ausspült, gegebenenfalls mit einem Shampoo wäscht und abschliessend trocknet.

Die Anwendung des Färbemittels mit zusätzlicher Festigung erfolgt in bekannter und üblicher Weise durch Befeuchten des Haares mit dem Festiger, Festlegen (Einlegen) der Haare zur Frisur und anschliessende Trocknung.

Die die 7-Nitro-2,1,3-benzoxadiazol-Derivate oder 7-Nitro-2,1,3-benzthiadiazol-Derivate der allgemeinen Formel (I) enthaltenden Färbemittel ermöglichen eine hervorragende, gleichmässige, intensive und gegen Shamponieren, Licht und Schweiß äußerst dauerhafte Färbung von Keratinfasern (insbesondere menschlichen Haaren) unter schonenden und hautverträglichen Bedingungen.

Die nachfolgenden Beispiele sollen den Gegenstand der Erfindung näher erläutern, ohne diesen hierauf zu beschränken.

### Beispiele

### Beispiel 1: Synthese von 4-(Dicyanomethyl)-7-nitro-2,1,3-benzoxadiazol-natriumsalz

0,5 g (2,5 mmol) 4-Chlor-7-nitro-2,1,3-benzoxadiazol werden in 13 ml Ethanol suspendiert. Dazu gibt man 0,265 g (2,5 mmol) Natriumcarbonat. Das Reaktionsgemisch wird bei Raumtemperatur (20-25 °C) mit 0,165 g (2,5 mmol) Malonsäuredinitril versetzt. Nach dreistündigem Erhitzen bei 50 °C wird das Reaktionsgemisch am Rotationsverdampfer aufkonzentriert, mit Aceton versetzt und filtriert. Das Filtrat wird am Rotationsverdampfer unter Vakuum eingeengt, wobei das gewünschte Produkt als Hydrat mit einem Mol Wasser ausfällt.
Die Ausbeute beträgt 95% der Theorie.
Schmelzpunkt: >300 °C
ESI (neg.)-Massenspektrum: M⁻-Na: 228 (100% rel. Intensität)
¹H-NMR (DMSO-D6, 500 MHz): δ = 8,18 ppm (d; J = 9,1 Hz; 1 H; H-C(6)); 6,48 ppm (d; J= 9,1 Hz; 1 H; H-C(5))
¹³C-NMR (DMSO-D6, 75.4 MHz): δ = 146,3 ppm (C(2)); 144,2 ppm (C(3)); 142,1 ppm (C(4)); 133,8 ppm (C(6)); 118,3 ppm (CN); 118,1 ppm (CN); 108,1 ppm (C(5)); 50,7 ppm (C(7))
UV-Vis Spektrum (EtOH): λₘₐₓ = 570 nm (42647); 382 nm (8300); 254 nm (8763)

**Elementaranalyse: C₉H₂N₅O₃ * Na* H₂O (269,16)**

| | %C | %H | %N |
|---|---|---|---|
| ber.: | 40,16 | 1,50 | 26,02 |
| gef.: | 40,16 | 1,76 | 25,19 |

### Beispiel 2: Synthese von 4-(1-Cyano-2-ethoxy-2-oxoethyl)-7-nitro-2,1,3-benzoxadiazol-natriumsalz

1,0 g (5 mmol) 4-Chlor-7-nitro-2,1,3-benzoxadiazol werden in 25 ml Ethanol suspendiert. Dazu gibt man 0,53 g (5 mmol) Natriumcarbonat. Das Reaktionsgemisch wird bei Raumtemperatur (20-25 °C) mit 0,57 g (5 mmol) Cyanessigsäureethylester versetzt. Nach sechsstündigem Erhitzen bei 50 °C wird das Reaktionsgemisch am Rotationsverdampfer aufkonzentriert, mit Aceton versetzt und filtriert. Anschliessend wird das Filtrat am Rotationsverdampfer unter Vakuum eingeengt.
Die Ausbeute beträgt 98% der Theorie.
Schmelzpunkt: 260°C (dc)
ESI (neg.)-Massenspektrum: M⁻-Na: 275 (100% rel. Intensität)
¹H-NMR (DMSO-D6, 500 MHz): δ = 8,11 ppm (d; J = 9,3 Hz; 1 H; H-C(6)); 7,89 ppm (d; J= 9,3 Hz; 1 H; H-C(5)); 4,15 ppm (q; J = 7,0 Hz; 2 H; CH₂); 1,24 ppm (t; J = 7,0 Hz; 3 H; CH₃)
UV-Vis Spektrum (EtOH): λₘₐₓ = 575 nm (38755); 389 nm (6539); 226 nm (8526)

**Elementaranalyse: C₁₁H₈N₄O₅ *Na (298,19)**

| | %C | %H | %N |
|---|---|---|---|
| ber.: | 44,31 | 2,37 | 18,79 |
| gef.: | 44,17 | 2,81 | 18,02 |

### Beispiel 3: Synthese von 4-(Dicyanomethyl)-7-nitro-2,1,3-benzoxadiazol-N-oxid-natriumsalz

0,91 g (5 mmol) 4-Nitrobenzofurazan-3-oxid werden in 15 ml Ethanol suspendiert. Dazu gibt man 1,06 g (10 mmol) Natriumcarbonat. Das Reaktionsgemisch wird bei Raumtemperatur (20-25 °C) mit 0,33 g (5 mmol) Malonsäuredinitril versetzt. Nach dreistündigem Erhitzen bei 50 °C wird das Reaktionsgemisch am Rotationsverdampfer aufkonzentriert, mit Aceton versetzt und filtriert. Das Filtrat wird sodann am Rotationsverdampfer im Vakuum eingeengt. Nach Chromatographie an Kieselgel (Essigsäureethylester / Methanol 9:1) erhält man 0,41 g des gewünschten Produktes in Form eines dunkelvioletten Pulvers.
Die Ausbeute beträgt 31 % der Theorie.
Schmelzpunkt: >250 °C
ESI (neg.)-Massenspektrum: M⁻-Na -O: 228 (100% rel. Intensität)
¹H-NMR (DMSO-D6, 500 MHz): δ = 8,21 ppm (d; J = 8,8 Hz; 1 H; H-C(6)); 6,51 ppm (d; J= 8,8 Hz; 1 H; H-C(5))
UV-Vis Spektrum (EtOH): λₘₐₓ = 574 nm (34163); 384 nm (7485); 250 nm (9425)

### Beispiel 4: Synthese von 4-(Dicyanomethyl)-7-nitro-2,1,3-benzthiadiazol-natriumsalz

0.91 g (5 mmol) 4-Nitro-2,1,3-benzothiadiazol werden in 15 ml Ethanol gelöst. Dazu gibt man 0,53 g (5 mmol) Natriumcarbonat. Das Reaktionsgemisch wird bei Raumtemperatur (20-25 °C) mit 0,33 g (5 mmol) Malonsäuredinitril versetzt. Nach siebenstündigem Erhitzen bei 50 °C wird das Reaktionsgemisch am Rotationsverdampfer aufkonzentriert, mit Methanol versetzt und filtriert. Anschliessend wird das Filtrat am Rotationsverdampfer unter Vakuum eingeengt. Nach Chromatographie an Kieselgel (Essigsäureethylester) erhält man 0,4 g des dunkelvioletten Produktes.
Die Ausbeute beträgt 33% der Theorie.
Schmelzpunkt: 230 °C
ESI (neg.)-Massenspektrum: M⁻-Na: 244 (100% rel. Intensität)
¹H-NMR (DMSO-D6, 500 MHz): δ = 8,33 ppm (d; J = 9,1 Hz; 1 H; H-C(6)); 6,71 ppm (d; J= 9,1 Hz; 1 H; H-C(5))
UV-Vis Spektrum (EtOH): λₘₐₓ = 574 nm (24028); 404 nm (7400); 312 nm (5040)

### Beispiel 5 bis 13: Synthese von 7-Nitro-2,1,3-benzoxadiazol-oder 7-Nitro-2,1,3-benzthiadiazol Derivaten

### Allgemeine Synthesevorschrift

Eine äquimolare Mischung aus 7-Nitro-2,1,3-benzoxadiazol beziehungsweise 7-Nitro-2,1,3-benzthiadiazol-Derivates der Formel (I) und der CH-aktiven Verbindung werden in Ethanol suspendiert und mit 1 bis 2 Äquivalenten Natriumcarbonat versetzt. Nach 3stündigem Erhitzen auf 50°C wird das Reaktionsgemisch am Rotationsverdampfer aufkonzentriert, mit Aceton versetzt, filtriert und das Filtrat am Rotationsverdampfer unter Vakuum eingeengt. Dabei fällt das gewünschte Produkt aus. Nach Reinigung durch Chromatographie an Kieselgel oder durch Umkristallisation wird das gewünschte Produkt in kristalliner Form isoliert.

In der nachfolgenden Tabelle 1 sind die entsprechenden Daten zusammengefasst.

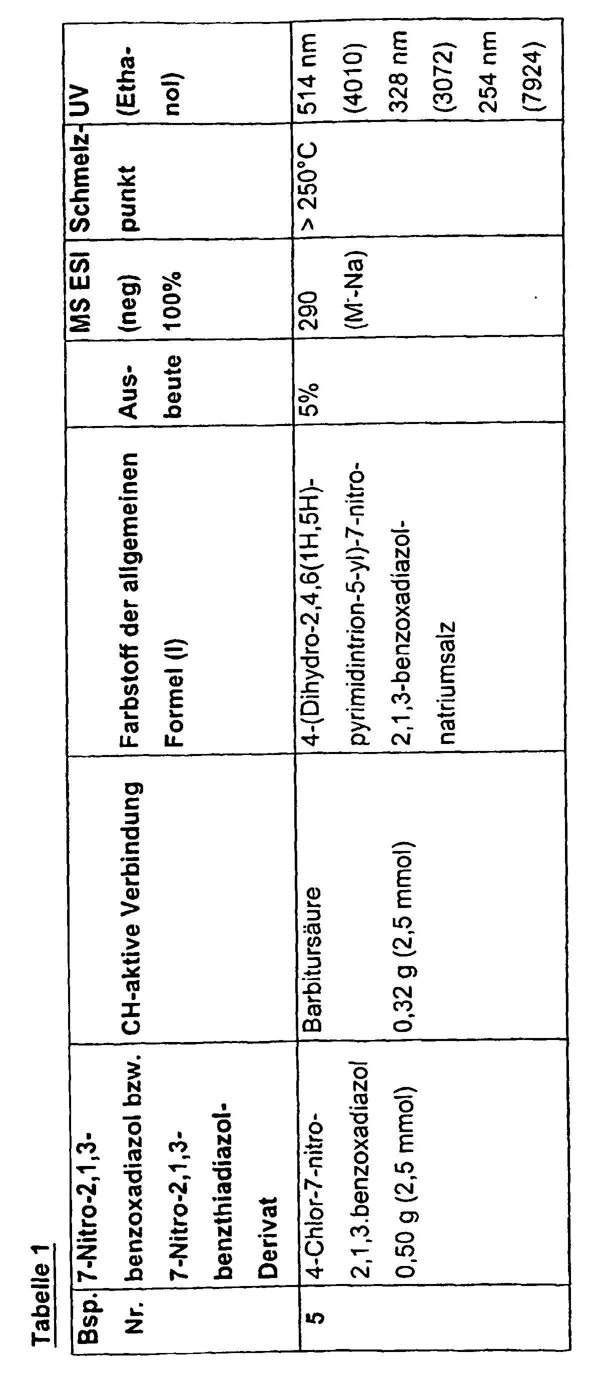

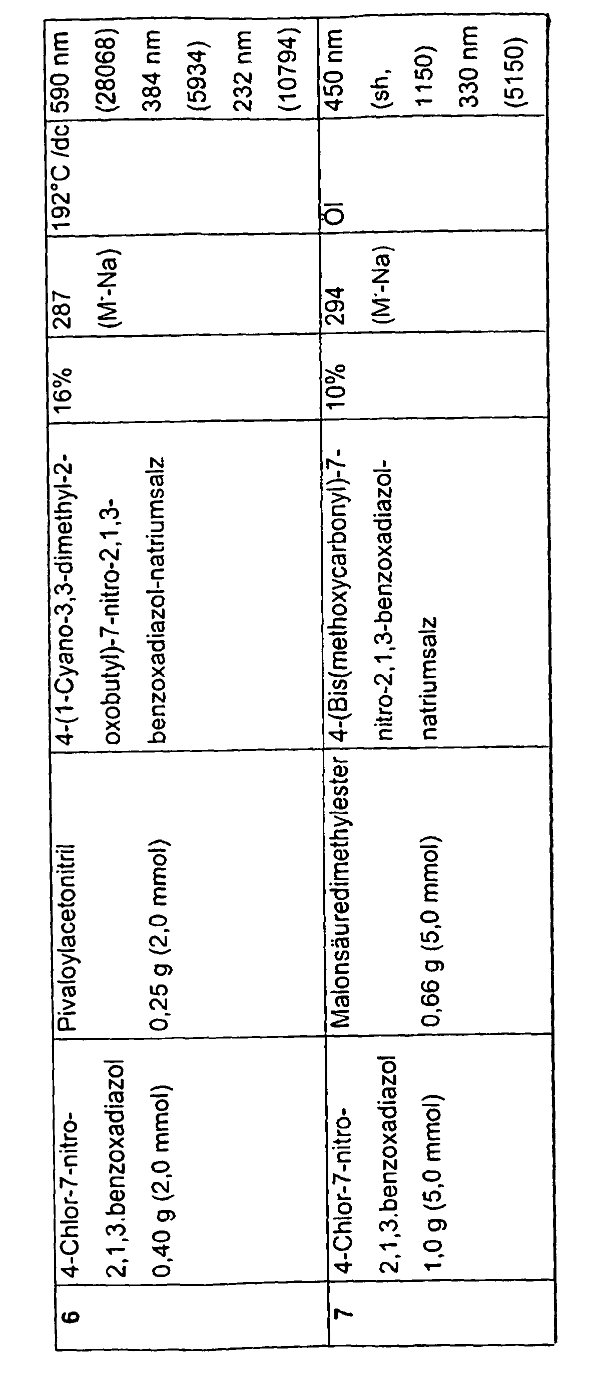

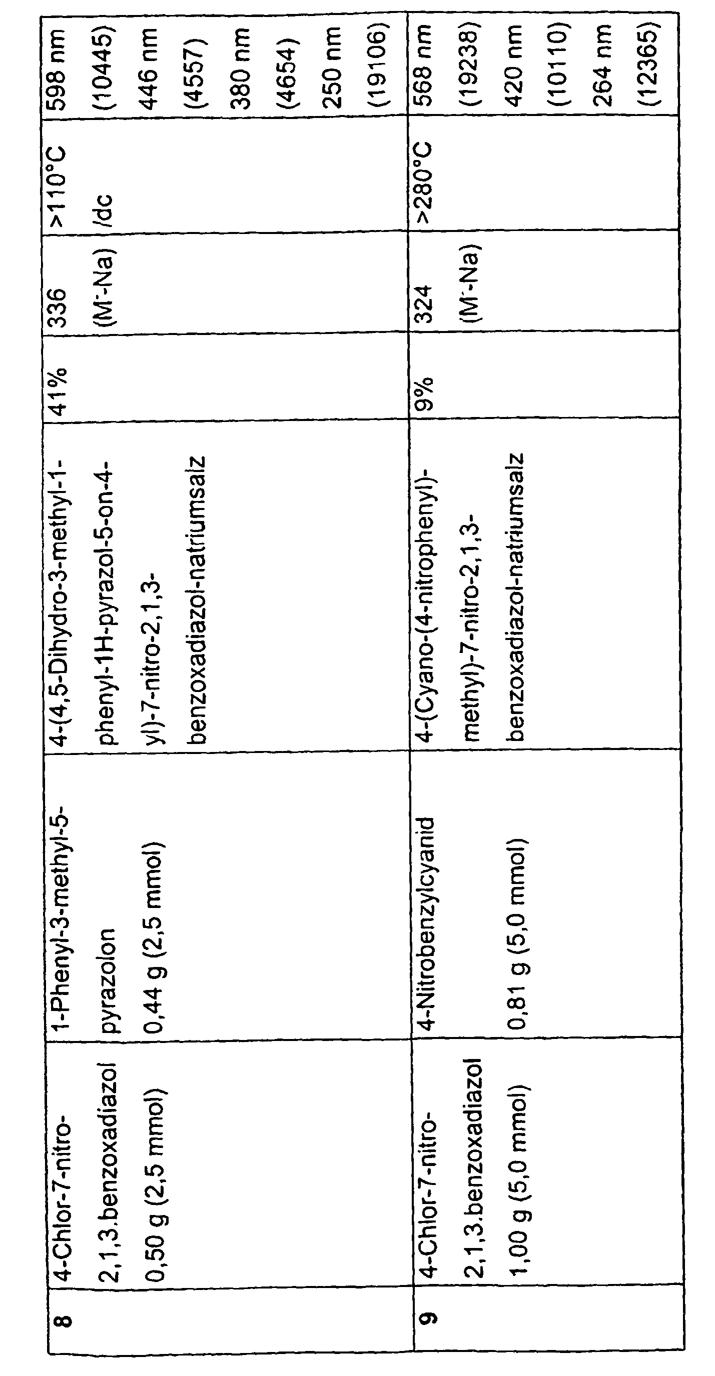

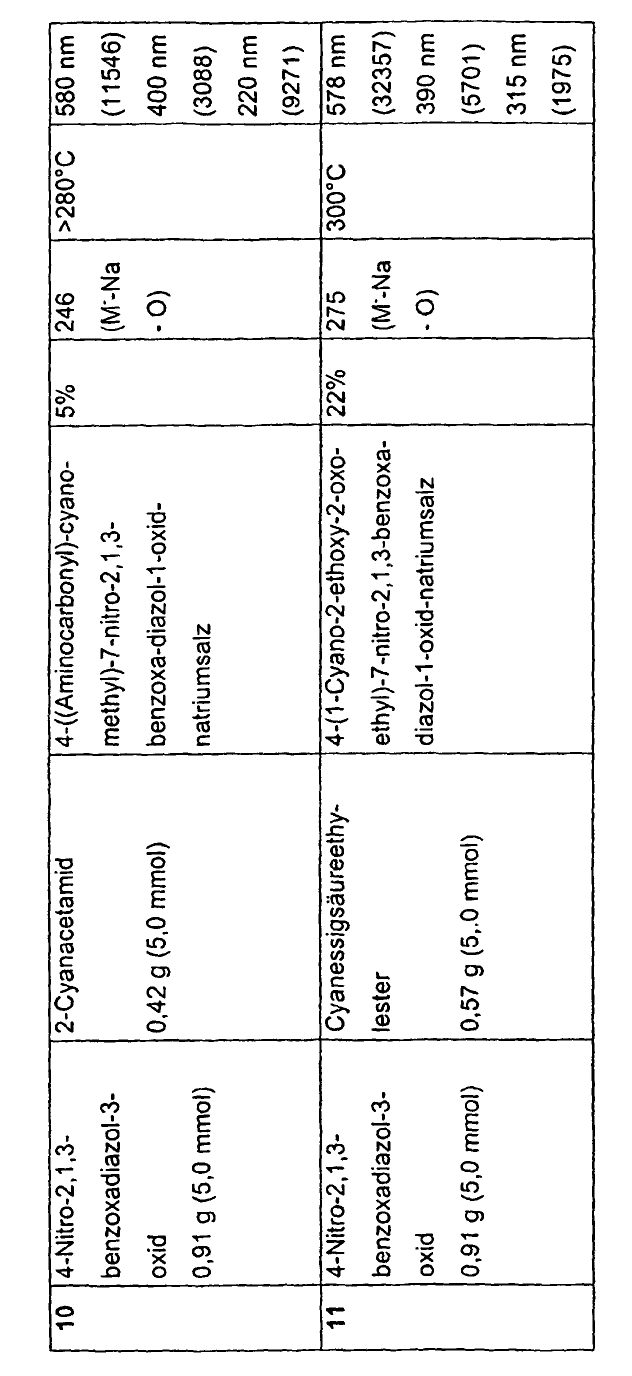

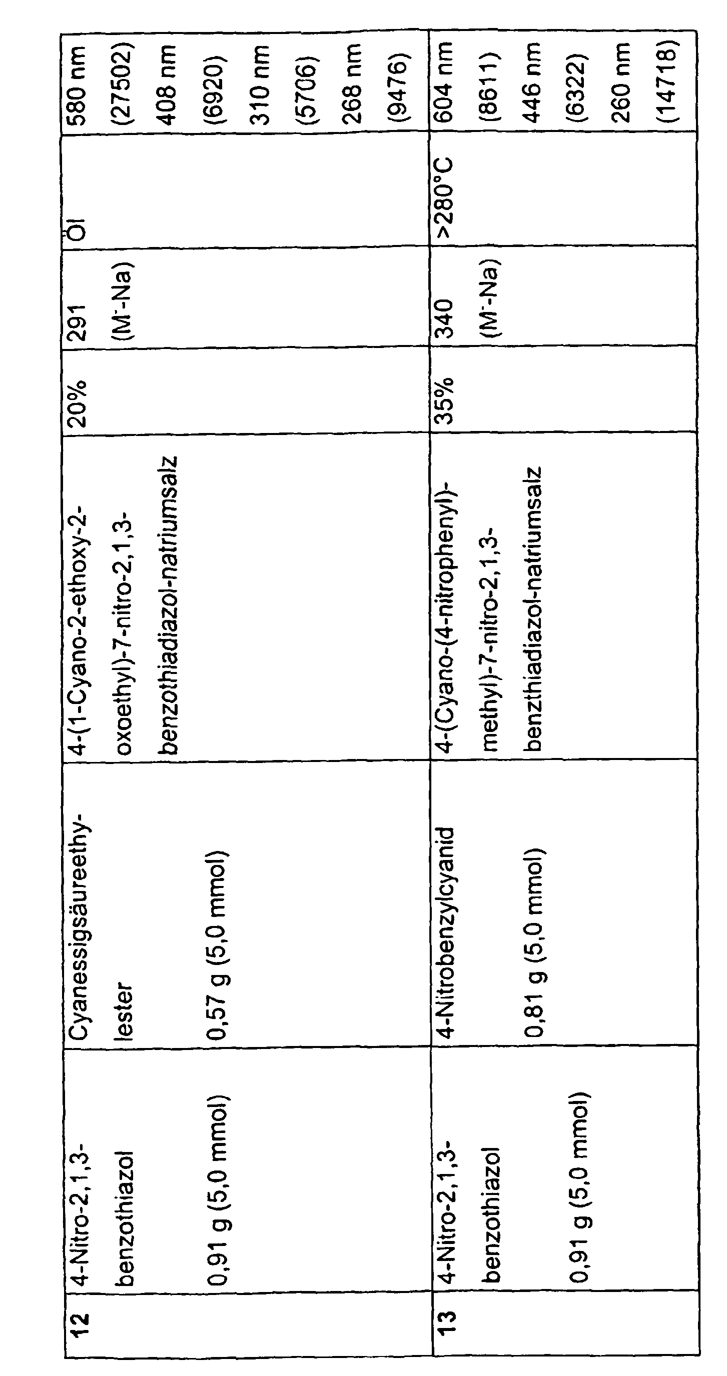

### Beispiele 14 bis 28: Haarfärbemittel

| | |
|---|---|
| 2,5 mmol | Farbstoff der Formel (I) gemäß Tabelle 2 |
| 5,0 g | Ethanol |
| 2,0 g | Decyl glucoside (Plantaren® 2000 UP NP) |
| 0,2 g | Ethylendiaminotetraessigsäure-Dinatriumsalz-Hydrat |
| ad 100,0 g | Wasser, vollentsalzt |

Die vorstehende Färbelösung wird durch Zugabe von Ammoniak oder Zitronensäure auf den in Tabelle 2 genannten pH-Wert eingestellt.

Die Haarfärbung erfolgt indem eine für die Haarfärbung ausreichende Menge des Färbemittels auf das Haar aufgetragen wird. Nach einer Einwirkungszeit von 30 Minuten bei 40 °C wird das Haar mit lauwarmem Wasser ausgespült und getrocknet.

Die Färbeergebnisse sind in der nachfolgenden Tabelle 2 zusammengefasst.

Die in den Beispielen angegbenen L*a*b*-Farbmeßwerte wurden mit einem Farbmessgerät der Firma Minolta, Typ Chromameter II, ermittelt. Hierbei steht der L-Wert für die Helligkeit (das heißt je geringer der L-Wert ist, umso grösser ist die Farbintensität), während der a-Wert ein Maß für den Rotanteil ist (das heißt je grösser der a-Wert ist, umso grösser ist der Rotanteil). Der b-Wert ist ein Maß für den Blauanteil der Farbe, wobei der Blauanteil umso größer ist, je negativer der b-Wert ist.

**Tabelle 2**

| **Bsp. Nr.** | **Farbstoff der Formel (I)** | **pH-Wert** | **Färbung** | **L*a*b*-Farbmesswerte** |
|---|---|---|---|---|
| **14** | 4-(Dicyanomethyl)-7-nitro- 2,1,3-benzoxadiazol-natriumsalz (gemäß Beispiel **1**) | 7,3 | tiefes violett | L= +25,17 |
| | | | | a= +54,12 |
| | | | | b= -24,03 |
| **15** | 4-(Dicyanomethyl)-7-nitro- 2,1,3-benzoxadiazol-natriumsalz (gemäß Beispiel **1**) mit Kupfer(II)chlorid | 3,8 | tiefes violett | L= +22,16 |
| | | | | a= +25,99 |
| | | | | b= -11,15 |
| **16** | 4-(1-Cyano-2-ethoxy-2-oxoethyl)-7-nitro-2,1,3-benzoxadiazol-natriumsalz (gemäß Beispiel **2**) | 5,4 | violett | L= +27,57 |
| | | | | a= +27,27 |
| | | | | b= + 3,22 |
| **17** | 4-(Dicyanomethyl)-7-nitro-2,1,3-benzoxadiazol-N-oxid-natriumsalz (gemäß Beispiel **3**) | 7,2 | blau-violett | L= +18,07 |
| | | | | a= +26,87 |
| | | | | b= -14,93 |
| **18** | 4-(Dicyanomethyl)-7-nitro-2,1,3-benzthiadiazol-natriumsalz (gemäß Beispiel **4**) | 7,2 | pink-violett | L= +38,94 |
| | | | | a= +50,44 |
| | | | | b= -28,02 |
| **19** | 4-(Dihydro-2,4,6(1H,5H)-pyrimidintrion-5-yl)-7-nitro-2,1,3-benzoxadiazol-natriumsalz (gemäß Beispiel **5**) | 3,0 | rot | L = +28,15 |
| | | | | a = +34,22 |
| | | | | b = +3,31 |
| **20** | 4-(1-Cyano-3,3-dimethyl-2-oxobutyl)-7-nitro-2,1,3-benzoxadiazol-natriumsalz (gemäß Beispiel **6**) | 10,0 | blau | L = +20,82 |
| | | | | a = +17,86 |
| | | | | b = -15,88 |
| **21** | 4-(Bis(methoxycarbonyl)-7-nitro-2,1,3-benzoxadiazol-natriumsalz (gemäß Beispiel **7**) | 6,5 | orange-rot | L = +37,82 |
| | | | | a = +41,64 |
| | | | | b = +22,80 |
| **22** | 4-(4,5-Dihydro-3-methyl-1-phenyl-1 H-pyrazol-5-on-4-yl)-7-nitro-2,1,3-benzoxadiazol- | 10,2 | türkis-blau | L = +27,15 |
| | | | | a = -4,60 |
| | natriumsalz (gemäß Beispiel **8**) | | | b = -16,72 |
| **23** | 4(Cyano-(4-nitrophenyl)-methyl)-7-nitro-2,1,3-benzoxadiazol-natriumsalz (gemäß Beispiel **9**) | 4,85 | braun-grau | L = +43,2 |
| | | | | a = +7,65 |
| | | | | b = +0,09 |
| **24** | 4-((Aminocarbonyl)-cyano-methyl)-7-nitro-2,1,3-benzoxa-diazol-1-oxid-natriumsalz (gemäß Beispiel **10**) | 6,4 | violett | L = +23,35 |
| | | | | a = +36,90 |
| | | | | b = -2,83 |
| **25** | 4-((Aminocarbonyl)-cyano-methyl)-7-nitro-2,1,3-benzoxadiazol-1-oxid-natriumsalz (gemäß Beispiel **10**) | 2,4 | violett-schwarz | L = +18,61 |
| | | | | a = +19,49 |
| | | | | b = +0,79 |
| **26** | 4-(1-Cyano-2-ethoxy-2-oxo-ethyl)-7-nitro-2,1,3-benzoxadiazol-1-oxid-natriumsalz (gemäß Beispiel **11**) | 6,6 | blau-violett | L = +22,68 |
| | | | | a = +38,55 |
| | | | | b = -19,99 |
| **27** | 4-(1-Cyano-2-ethoxy-2-oxo- ethyl)-7-nitro-2,1,3-benzthiadiazol-natriumsalz (gemäß Beispiel **12**) | 7,0 | blau-violett | L = +22,67 |
| | | | | a = +27,77 |
| | | | | b = -20,98 |
| **28** | 4-(Cyano-(4-nitrophenyl)-methyl)-7-nitro-2,1,3-benzthiadiazol-natriumsalz (gemäß Beispiel **13**) | 4,7 | hellgrau | L = +47,5 |
| | | | | a = -2,29 |
| | | | | b = +6,15 |

Alle in der vorliegenden Anmeldung genannten Prozentangaben stellen soweit nicht anders angegeben Gewichtsprozente dar.

## Patentansprüche

1. 7-Nitro-2,1,3-benz-oxadiazol-Derivate und 7-Nitro-2,1,3-benzthiadiazol-Derivate der allgemeinen Formel (I) 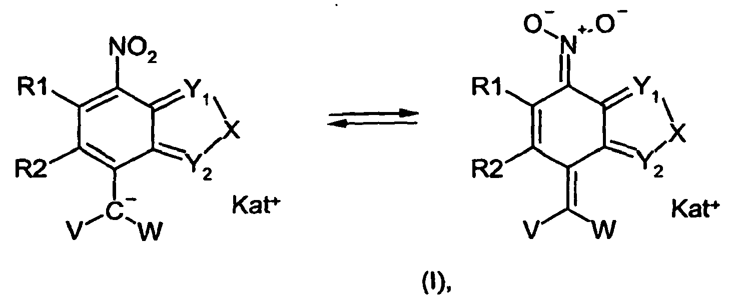
wobei gilt:
**X** ist gleich Sauerstoff oder Schwefel;
**Y1** und **Y2** können gleich oder verschieden sein und stehen unabhängig voneinander für ein Stickstoffatom oder eine Stickstoffmonoxid-Gruppe (NO);
**R1** und **R2** können gleich oder verschieden sein und stellen unabhängig voneinander Wasserstoff, ein Halogenatom, eine (C₁-C₄)-Alkylgruppe, eine mit einem Halogenatom substituierte (C₁-C₄)-Alkylgruppe, eine (C₁-C₄)-Alkoxygruppe, eine Nitrogruppe oder eine NR^{a}R^{b}-Gruppe dar, wobei die Reste R^{a} und R^{b} gleich oder verschieden sein können und unabhängig voneinander Wasserstoff, eine (C₁-C₄)-Alkyl-gruppe, einen gegebenfalls substituierten aromatischen Carbozyklus oder eine (C₁-C₄)-Alkancarbonylgruppe darstellen, oder R^{a} und R^{b} gemeinsam mit dem Stickstoffatom einen heterozyklischen (C₃-C₆)- Rest bilden;
V ist gleich Wasserstoff, einem aliphatischen Rest, einem aromatischen isozyklischen Rest, einem aromatischen heterozyklischen Rest, einer Cyanogruppe oder einer Carbonylfunktion (CO)-R3, wobei R3 für Wasserstoff, eine Hydroxygruppe, eine (C₁-C₄)-Alkoxygruppe, eine Aminogruppe, eine (C₁-C₄)-Alkylaminogruppe, eine (C₁-C₆)-Alkylgruppe oder eine Arylgruppe steht;
**W** stellt eine Cyanogruppe oder eine Carbonylfunktion (CO)-R4 dar, wobei R4 für Wasserstoff, eine Hydroxygruppe, eine (C₁-C₄)-Alkoxygruppe, eine Aminogruppe, eine (C₁-C₄)-Alkylaminogruppe, eine (C₁-C₆)-Alkylgruppe oder eine Arylgruppe steht; alternativ können **V** und **W** auch gemeinsam ein aliphatisches oder aromatisches isozyklisches oder heterozyklisches Ringsystem bilden; und
**Kat**^{**+**} entspricht einem Alkalikation, einem Erdalkalikation, einer quaternären Ammoniumgruppe, einer quaternären Phosphoniumgruppe oder einer Sulfoniumgruppe;
unter der Bedingung, dass **W** nicht gleich einer CO-Phenylgruppe, einer CO-(4-Bromphenyl-)-Gruppe, einer CO-(4-Chlorphenyl-)-Gruppe, einer CO-(4-Methylphenyl-)-Gruppe oder einer CO-(4-Methoxyphenyl-)Gruppe ist, wenn **R1** gleich Wasserstoff ist, **V** gleich Wasserstoff oder einem unsubstituierten Pyridylrest ist, **R2** gleich einer Nitrogruppe ist, **X** gleich Sauerstoff ist und **Y1** und **Y2** gleich einem Stickstoffatom sind.

2. Verbindungen gemäß Anspruch 1, **dadurch gekennzeichnet, dass** gilt: **X** ist gleich Sauerstoff oder Schwefel ;
**Y1** und **Y2** können gleich oder verschieden sein und stehen unabhängig voneinander für ein Stickstoffatom oder eine Stickstoffmonoxid-Gruppe (NO);
**R1** und **R2** können gleich oder verschieden sein und stellen unabhängig voneinander Wasserstoff, ein Halogenatom, eine (C1-C4)-Alkylgruppe oder eine Nitrogruppe dar;
**V** ist gleich Wasserstoff, einem aliphatischen Rest, einem aromatischen isozyklischen Rest, einem aromatischen heterozyklischen Rest, einer Cyanogruppe oder einer Carbonylfunktion (CO)-R3, wobei R3 für Wasserstoff, eine Hydroxygruppe, eine (C₁-C₄)-Alkoxygruppe, eine Aminogruppe, eine (C₁-C₄)-Alkylaminogruppe, eine (C₁-C₆)-Alkylgruppe oder eine Arylgruppe steht;
**W** stellt eine Cyanogruppe oder eine Carbonylfunktion (CO)-R4 dar, wobei R4 für Wasserstoff, eine Hydroxygruppe, eine (C₁-C₄)-Alkoxygruppe, eine Aminogruppe, eine (C₁-C₄)-Alkylaminogruppe, eine (C₁-C₆)-Alkylgruppe oder eine Arylgruppe steht; alternativ können **V** und **W** auch gemeinsam ein aliphatisches oder aromatisches isozyklisches oder heterozyklisches Ringsystem bilden; und
**Kat**^{**+**} entspricht einem Alkalikation, einem Erdalkalikation, einer quaternären Ammoniumgruppe, einer quaternären Phosphoniumgruppe oder einer Sulfoniumgruppe;
unter der Bedingung, dass **W** nicht gleich einer CO-Phenylgruppe, einer CO-(4-Bromphenyl-)-Gruppe, einer CO-(4-Chlorphenyl-)-Gruppe, einer CO-(4-Methylphenyl-)-Gruppe oder einer CO-(4-Methoxyphenyl-)Gruppe ist, wenn **R1** gleich Wasserstoff ist, **V** gleich Wasserstoff oder einem unsubstituierten Pyridylrest ist, **R2** gleich einer Nitrogruppe ist, **X** gleich Sauerstoff ist und **Y1** und **Y2** gleich einem Stickstoffatom sind.

3. Verbindung gemäß Anspruch 1 oder 2, **dadurch gekennzeichnet, dass** das 7-Nitro-2,1,3-benzoxadiazol-Derivat der Formel (I) ausgewählt ist aus 4-(Dicyanomethyl)-7-nitro-2,1,3-benzoxadiazol-natnumsalz, 4-(1-Cyano-2-ethoxy-2-oxoethyl)-7-nitro-2,1,3-benzoxadiazol-natriumsalz, 4-(Dicyanomethyl)-7-nitro-2,1,3-benzoxadiazol-N-oxid-natriumsalz, 4-(Dihydro-2,4,6(1H,5H)-pyrimidintrion-5-yl)-7-nitro-2,1,3-benzoxadiazol-natriumsalz, 4-(1-Cyano-3,3-dimethyl-2-oxobutyl)-7-nitro-2,1,3-benzoxadiazol-natriumsalz, 4-(Bis(methoxycarbonyl)-7-nitro-2,1,3-benzoxadiazol-natriumsalz, 4-(4,5-Dihydro-3-methyl-1-phenyl-1H-pyrazol-5-on-4-yl)-7-nitro-2,1,3-benzoxadiazol-natriumsalz, 4-(Cyano-(4-nitro-phenyl)-methyl)-7-nitro-2,1,3-benzoxadiazol-natriumsalz, 4-((Amino-carbonyl)-cyano-methyl)-7-nitro-2,1,3-benzoxa-diazol-1-oxid-natriumsalz, 4-(1-Cyano-2-ethoxy-2-oxo-ethyl)-7-nitro-2,1,3-benzoxa-diazol-1-oxidnatriumsalz, 4-(1,3-Cyclohexandion-2-yl)-7-nitro2,1,3-benzoxadiazol-natriumsalz, 4-(Carboxy-cyanomethyl)-7-nitro-2,1,3-benzoxadiazol-natriumsalz, 4-(2-Ethoxy-1-nitro-2-oxoethyl)-7-nitro-2,1,3-benzoxadiazol-natriumsalz, 4-((Aminocarbonyl)cyanomethyl)-7-nitro-2,1,3-benzoxadiazol-natriumsalz, 4-(Dihydro-2-thioxo-4,6(1H,5H)-pyrimidindion-5-yl)-7-nitro-2,1,3-benzoxadiazol-1-oxid-natriumsalz, 4-(1,3-Dioxo-indan-2-yl)-7-nitro-2,1,3-benzoxadiazol-natriumsalz, 4-(2-Oxo-2,3-dihydro-1H-indol-3-yl)-7-nitro-2,1,3-benzoxadiazol-natriumsalz, 4-(4-Oxo-2-thioxo-thiazolidin-5-yl)-7-nitro-2,1,3-benzoxadiazol-natriumsalz, 4-(Dihydro-6-thioxo-2,4-(1H,5H)-pyrimidin-dion-3-yl)-7-nitro-2,1,3-benzoxadiazol-natriumsalz, 4-(1-Cyano-2-oxo-2-phenylethyl)-2,1,3-benzoxadiazol-natriumsalz und 4-(Cyano-(2-nitrophenyl)-methyl)-7-nitro-2,1,3-benzoxadiazol-natriumsalz.

4. Verbindung gemäß Anspruch 1 oder 2, **dadurch gekennzeichnet, dass** das 7-Nitro-2,1,3-benzthiadiazol-Derivat der Formel (I) ausgewählt ist aus 4-(Dicyanomethyl)-7-nitro-2,1,3-benzthiadiazol-natriumsalz, 4-(1-Cyano-2-ethoxy-2-oxoethy-2-nitro-2,1,3-benzo-thiadiazolnatriumsalz, 4-(Cyano-(4-nitrophenyl)methyl)-7-nitro-2,1,3-benzthiadiazol-natriumsalz, 4-(Dicyanomethyl)-7-nitro-2,1,3-benz-thiadiazol-N-oxidnatriumsalz, 4-(Dihydro-2,4,6(1H,5H)-pyrimidintrion-5-yl)-7-nitro-2,1,3-benzthiadiazol-natriumsalz, 4-(1-Cyano-3,3-dimethyl-2-oxobutyl)-7-nitro-2,1,3-benzthiadiazol-natriumsalz, 4-(Bis(methoxy-carbonyl)-7-nitro-2,1,3-benzthiadiazol-natriumsalz, 4-(4,5-Dihydro-3-methyl-1-phenyl-1H-pyrazol-5-on-4-yl)-7-nitro-2,1,3-benzthiadiazol-natriumsalz, 4-(1,3-Cyclohexandion-2-yl)-7-nitro-2,1,3-benzthiadiazol-natriumsalz, 4-(Carboxy-cyanomethyl)-7-nitro-2,1,3-benzthiadiazol-natriumsalz, 4-(2-Ethoxy-1-nitro-2-oxoethyl)-7-nitro-2,1,3-benzthiadiazol-natriumsalz, 4-((Aminocarbonyl)-cyanomethyl)-7-nitro-2,1,3-benzthiadiazol-natriumsalz, 4-(Dihydro-2-thioxo-4,6(1H,5H)-pyrimidindion-5-yl)-7-nitro-2,1,3-benz-thiadiazol-1-oxidnatriumsalz, 4-(1,3-Dioxo-indan-2-yl)-7-nitro-2,1,3-benzthiadiazol-natriumsalz, 4-(2-Oxo-2,3-dihydro-1H-indol-3-yl)-7-nitro-2,1,3-benzthiadiazol-natriumsalz, 4-(4-Oxo-2-thioxo-thiazolidin-5-yl)-7-nitro-2,1,3-benzthiadiazol-natriumsalz, 4-(Dihydro-6-thioxo-2,4,(1H,5H)-pyrimidin-dion-3-yl)-7-nitro-2,1,3-benzthiadiazol-natriumsalz, 4-(1-Cyano-2-oxo-2-phenylethyl)-2,1,3-benzthiadiazol-natriumsalz und 4-(Cyano-(2-nitrophenyl)-methyl)-7-nitro-2,1,3-benzoxadiazol-natriumsalz.

5. Mittel zur Färbung von Keratinfasern, **dadurch gekennzeichnet, dass** es mindestens ein 7-Nitro-2,1,3-benzoxadiazol-Derivat und/oder 7-Nitro-2,1,3-benzthiadiazol-Derivat der allgemeinen Formel (I) nach einem der Ansprüche 1 bis 4 enthält.

6. Mittel nach Anspruch 5, **dadurch gekennzeichnet, dass** es die Verbindung der Formel (I) in einer Menge von 0,01 bis 10 Gewichtsprozent enthält.

7. Mittel nach Anspruch 5 oder 6, **dadurch gekennzeichnet, dass** es zusätzlich zu der Verbindung der Formel (I) mindestens einen weiteren direktfärbenden Farbstoff aus der Gruppe der anionischen, kationischen, nicht-ionischen oder amphoteren Farbstoffe, Nitrofarbstoffe, Azofarbstoffe, Anthrachinonfarbstoffe und Dispersionsfarbstoffe enthält.

8. Mittel nach einem der Ansprüche 5 bis 7, **dadurch gekennzeichnet, dass** es mindestens ein für kosmetische Mittel übliches Polymer aus der Gruppe bestehend aus natürlichen oder synthetischen Polymeren und modifizierten Polymeren natürlichen Ursprungs enthält und in Form eines Tönungsfestigers oder Farbfestigers vorliegt.

9. Mittel nach einem der Ansprüche 5 bis 8, **dadurch gekennzeichnet, dass** es ein Haarfärbemittel ist.

10. Verwendung von 7-Nitro-2,1,3-benzoxadiazol-Derivaten und/oder 7-Nitro-2,1,3-benzthiadiazol-Derivaten der allgemeinen Formel (I) nach einem der Ansprüche 1 bis 4 als Farbstoff in Färbemitteln für Keratinfasern.

## Claims

1. 7-Nitro-2,1,3-benzoxadiazole derivatives and 7-nitro-2,1,3-benzothiadiazole derivatives of the general formula (I) 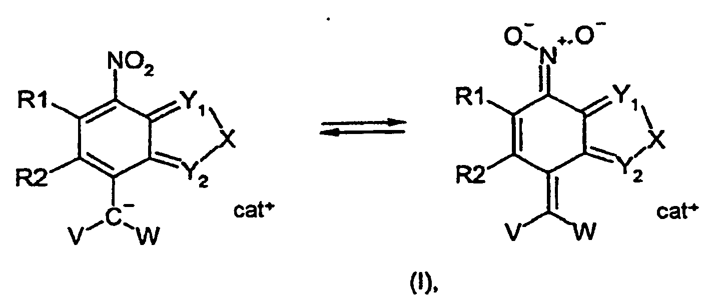
where:
**X** is oxygen or sulphur;
**Y1** and **Y2** may be identical or different and, independently of one another, are a nitrogen atom or a nitrogen monoxide group (NO);
**R1** and **R2** may be identical or different and, independently of one another, are hydrogen, a halogen atom, a (C₁-C₄)-alkyl group, a (C₁-C₄)-alkyl group substituted by a halogen atom, a (C₁-C₄)-alkoxy group, a nitro group or an NR^{a}R^{b} group, where the radicals R^{a} and R^{b} may be identical or different and, independently of one another, are hydrogen, a (C₁-C₄)-alkyl group, an optionally substituted aromatic carbocycle or a (C₁-C₄)-alkanecarbonyl group, or R^{a} and R^{b}, together with the nitrogen atom, form a heterocyclic (C₃-C₆) radical;
**V** is hydrogen, an aliphatic radical, an aromatic isocyclic radical, an aromatic heterocyclic radical, a cyano group or a carbonyl function (CO)-R3, where R3 is hydrogen, a hydroxy group, a (C₁-C₄)-alkoxy group, an amino group, a (C₁-C₄)-alkylamino group, a (C₁-C₆)-alkyl group or an aryl group;
**W** is a cyano group or a carbonyl function (CO)-R4, where R4 is hydrogen, a hydroxy group, a (C₁-C₄)-alkoxy group, an amino group, a (C₁-C₄)-alkylamino group, a (C₁-C₆)-alkyl group or an aryl group; alternatively, **V** and **W** can also together form an aliphatic or aromatic isocyclic or heterocyclic ring system; and
**cat**^{**+**} is an alkali metal cation, an alkaline earth metal cation, a quaternary ammonium group, a quaternary phosphonium group or a sulphonium group;
with the proviso that **W** is not a CO-phenyl group, a CO-(4-bromophenyl) group, a CO-(4-chlorophenyl) group, a CO-(4-methylphenyl) group or a CO-(4-methoxyphenyl) group if **R1** is hydrogen, **V** is hydrogen or an unsubstituted pyridyl radical, **R2** is a nitro group, **X** is oxygen and **Y1** and **Y2** are a nitrogen atom.

2. Compounds according to Claim 1, **characterized in that**: **X** is oxygen or sulphur;
**Y1** and **Y2** may be identical or different and, independently of one another, are a nitrogen atom or a nitrogen monoxide group (NO);
**R1** and **R2** may be identical or different and, independently of one another, are hydrogen, a halogen atom, a (C1-C4)-alkyl group or a nitro group;
**V** is hydrogen, an aliphatic radical, an aromatic isocyclic radical, an aromatic heterocyclic radical, a cyano group or a carbonyl function (CO)-R3, where R3 is hydrogen, a hydroxy group, a (C₁-C₄)-alkoxy group, an amino group, a (C₁₋C₄)-alkylamino group, a (C₁-C₆)-alkyl group or an aryl group;
**W** is a cyano group or a carbonyl function (CO)-R4, where R4 is hydrogen, a hydroxy group, a (C₁-C₄)-alkoxy group, an amino group, a (C₁-C₄)-alkylamino group, a (C₁-C₆)-alkyl group or an aryl group; alternatively, V and **W** can also together form an aliphatic or aromatic isocyclic or heterocyclic ring system; and
**cat**^{**+**} is an alkali metal cation, an alkaline earth metal cation, a quaternary ammonium group, a quaternary phosphonium group or a sulphonium group;
with the proviso that **W** is not a CO-phenyl group, a CO-(4-bromophenyl) group, a CO-(4-chlorophenyl) group, a CO-(4-methylphenyl) group or a CO-(4-methoxyphenyl) group if **R1** is hydrogen, **V** is hydrogen or an unsubstituted pyridyl radical, **R2** is a nitro group, **X** is oxygen and **Y1** and **Y2** are a nitrogen atom.

3. Compound according to Claim 1 or 2, **characterized in that** the 7-nitro-2,1,3-benzoxadiazole derivative of the formula (I) is chosen from 4-(dicyanomethyl)-7-nitro-2,1,3-benzoxadiazole sodium salt, 4-(1-cyano-2-ethoxy-2-oxoethyl)-7-nitro-2,1,3-benzoxadiazole sodium salt, 4-(dicyanomethyl)-7-nitro-2,1,3-benzoxadiazole N-oxide sodium salt, 4-(dihydro-2,4,6(1H,5H)-pyrimidinetrion-5-yl)-7-nitro-2,1,3-benzoxadiazole sodium salt, 4-(1-cyano-3,3-dimethyl-2-oxobutyl)-7-nitro-2,1,3-benzoxadiazole sodium salt, 4-(bis(methoxycarbonyl)-7-nitro-2,1,3-benzoxadiazole sodium salt, 4-(4,5-dihydro-3-methyl-1-phenyl-1H-pyrazol-5-on-4-yl)-7-nitro-2,1,3-benzoxadiazole sodium salt, 4-(cyano-(4-nitrophenyl)methyl)-7-nitro-2,1,3-benzoxadiazole sodium salt, 4-((aminocarbonyl)cyanomethyl)-7-nitro-2,1,3-benzoxadiazole 1-oxide sodium salt, 4-(1-cyano-2-ethoxy-2-oxoethyl)-7-nitro-2,1,3-benzoxadiazole 1-oxide sodium salt, 4-(1,3-cyclohexanediol-2-yl)-7-nitro-2,1,3-benzoxadiazole sodium salt, 4-(carboxycyanomethyl)-7-nitro-2,1,3-benzoxadiazole sodium salt, 4-(2-ethoxy-1-nitro-2-oxoethyl)-7-nitro-2,1,3-benzoxadiazole sodium salt, 4-((aminocarbonyl)cyanomethyl)-7-nitro-2,1,3-benzoxadiazole sodium salt, 4-(dihydro-2-thioxo-4,6(1H,5H)-pyrimidinedion-5-yl)-7-nitro-2,1,3-benzoxadiazole 1-oxide sodium salt, 4-(1,3-dioxoindan-2-yl)-7-nitro-2,1,3-benzoxadiazole sodium salt, 4-(2-oxo-2,3-dihydro-1H-indol-3-yl)-7-nitro-2,1,3-benzoxadiazole sodium salt, 4-(4-oxo-2-thioxothiazolidin-5-yl)-7-nitro-2,1,3-benzoxadiazole sodium salt, 4-(dihydro-6-thioxo-2,4-(1H,5H)-pyrimidinedion-3-yl)-7-nitro-2,1,3-benzoxadiazole sodium salt, 4-(1-cyano-2-oxo-2-phenylethyl)-2,1,3-benzoxadiazole sodium salt and 4-(cyano(2-nitrophenyl)methyl)-7-nitro-2,1,3-benzoxadiazole sodium salt.

4. Compound according to Claim 1 or 2, **characterized in that** the 7-nitro-2,1,3-benzothiadiazole derivative of the formula (I) is chosen from 4-(dicyanomethyl)-7-nitro-2,1,3-benzothiadiazole sodium salt, 4-(1-cyano-2-ethoxy-2-oxoethyl)-7-nitro-2,1,3-benzothiadiazole sodium salt, 4-(cyano(4-nitrophenyl)methyl)-7-nitro-2,1,3-benzothiadiazole sodium salt, 4-(dicyanomethyl)-7-nitro-2,1,3-benzothiadiazole N-oxide sodium salt, 4-(dihydro-2,4,6-(1H,5H)-pyrimidinetrion-5-yl)-7-nitro-2,1,3-benzothiadiazole sodium salt, 4-(1-cyano-3,3-dimethyl-2-oxobutyl)-7-nitro-2,1,3-benzothiadiazole sodium salt, 4-(bis(methoxycarbonyl)-7-nitro-2,1,3-benzothiadiazole sodium salt, 4-(4,5-dihydro-3-methyl-1-phenyl-1H-pyrazol-5-on-4-yl)-7-nitro-2,1,3-benzothiadiazole sodium salt, 4-(1,3-cyclohexanedion-2-yl)-7-nitro-2,1,3-benzothiadiazole sodium salt, 4-(carboxycyanomethyl)-7-nitro-2,1,3-benzothiadiazole sodium salt, 4-(2-ethoxy-1-nitro-2-oxoethyl)-7-nitro-2,1,3-benzothiadiazole sodium salt, 4-((aminocarbonyl)cyanomethyl)-7-nitro-2,1,3-benzothiadiazole sodium salt, 4-(dihydro-2-thioxo-4,6-(1H,5H)pyrimidinedion-5-yl)-7-nitro-2,1,3-benzothiadiazole 1-oxide sodium salt, 4-(1,3-dioxoindan-2-yl)-7-nitro-2,1,3-benzothiadiazole sodium salt, 4-(2-oxo-2,3-dihydro-1H-indol-3-yl)-7-nitro-2,1,3-benzothiadiazole sodium salt, 4-(4-oxo-2-thioxothiazolidin-5-yl)-7-nitro-2,1,3-benzothiadiazole sodium salt, 4-(dihydro-6-thioxo-2,4-(1H,5H)-pyrimidinedion-3-yl)-7-nitro-2,1,3-benzothiadiazole sodium salt, 4-(1-cyano-2-oxo-2-phenylethyl)-2,1,3-benzothiadiazole sodium salt and 4-(cyano(2-nitrophenyl)methyl)-7-nitro-2,1,3-benzothiadiazole sodium salt.

5. Agent for dyeing keratin fibres, **characterized in that** it comprises at least one 7-nitro-2,1,3-benzoxadiazole derivative and/or 7-nitro-2,1,3-benzothiadiazole derivative of the general formula (I) according to one of Claims 1 to 4.

6. Agent according to Claim 5, **characterized in that** it comprises the compound of the formula (I) in an amount of from 0.01 to 10% by weight.

7. Agent according to Claim 5 or 6, **characterized in that**, in addition to the compound of the formula (I), it comprises at least one further direct dye from the group of anionic, cationic, non-ionic or amphoteric dyes, nitro dyes, azo dyes, anthraquinone dyes and dispersion dyes.

8. Agent according to one of Claims 5 to 7, **characterized in that** it comprises at least one polymer customary for cosmetic agents from the group consisting of natural or synthetic polymers and modified polymers of natural origin and is present in the form of a setting tint or setting colour.

9. Agent according to one of Claims 5 to 8, **characterized in that** it is a hair dyeing agent.

10. Use of 7-nitro-2,1,3-benzoxadiazole derivatives and/or 7-nitro-2,1,3-benzothiadiazole derivatives of the general formula (I) according to one of Claims 1 to 4 as dye in dyeing agents for keratin fibres.

## Revendications

1. Dérivés de 7-nitro-2,1,3-benzoxadiazole et dérivés de 7-nitro-2,1,3-benzthiadiazole de formule générale (I) 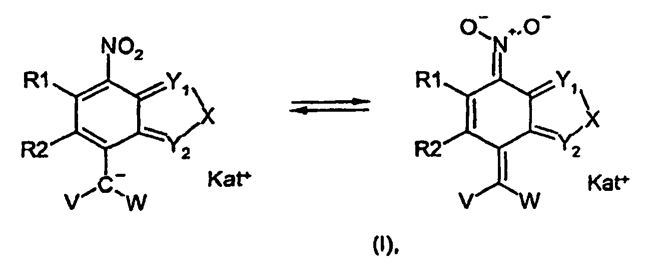
dans laquelle :
X représente un oxygène ou un soufre ;
Y1 et Y2 peuvent être identiques ou différents et représentent, indépendamment l'un de l'autre, un atome d'azote ou un groupe monoxyde d'azote (NO) ;
R1 et R2 peuvent être identiques ou différents et représentent, indépendamment l'un de l'autre, un hydrogène, un atome d'halogène, un groupe alkyle en C₁-C₄, un groupe alkyle en C₁-C₄ substitué par un atome d'halogène, un groupe alcoxy en C₁-C₄, un groupe nitro ou un groupe NR^{a}R^{b}, où les radicaux R^{a} et R^{b} peuvent être identiques ou différents et représentent, indépendamment l'un de l'autre, un hydrogène, un groupe alkyle en C₁-C₄, un carbocycle aromatique éventuellement substitué ou un groupe (C₁-C₄)alcanecarbonyle, ou R^{a} et R^{b}, conjointement avec l'atome d'azote, forment un radical en C₃-C₆ hétérocyclique ;
V représente un hydrogène, un radical aliphatique, un radical isocyclique aromatique, un radical hétérocyclique aromatique, un groupe cyano ou une fonction carbonyle (CO)-R3, dans laquelle R3 représente un hydrogène, un groupe hydroxy, un groupe alcoxy en C₁-C₄, un groupe amino, un groupe alkylamino en C₁-C₄, un groupe alkyle en C₁-C₆ ou un groupe aryle ;
W représente un groupe cyano ou une fonction carbonyle (CO)-R4, dans laquelle R4 représente un hydrogène, un groupe hydroxy, un groupe alcoxy en C₁-C₄, un groupe amino, un groupe alkylamino en C₁₋C₄, un groupe alkyle en C₁-C₆ ou un groupe aryle ; en variante, V et W peuvent également former conjointement un système de noyau aliphatique ou aromatique, isocyclique ou hétérocyclique ; et
Kat⁺ correspond à un cation de métal alcalin, à un cation de métal alcalino-terreux, à un groupe ammonium quaternaire, à un groupe phosphonium quaternaire ou à un groupe sulfonium ;
à condition que W ne représente pas un groupe CO-phényle, un groupe CO-(4-bromophényle), un groupe CO-(4-chlorophényle), un groupe CO-(4-méthylphényle) ou un groupe CO-(4-méthoxyphényle), quand R1 représente un hydrogène, V représente un hydrogène ou un radical pyridyle non substitué, R2 représente un groupe nitro, X représente un oxygène et Y1 et Y2 représentent un atome d'azote.

2. Composés selon la revendication 1, **caractérisés en ce que** : X représente un oxygène ou un soufre ;
Y1 et Y2 peuvent être identiques ou différents et représentent, indépendamment l'un de l'autre, un atome d'azote ou un groupe monoxyde d'azote (NO) ;
R1 et R2 peuvent être identiques ou différents et représentent, indépendamment l'un de l'autre, un hydrogène, un atome d'halogène, un groupe alkyle en C₁-C₄ ou un groupe nitro ;
V représente un hydrogène, un radical aliphatique, un radical isocyclique aromatique, un radical hétérocyclique aromatique, un groupe cyano ou une fonction carbonyle (CO)-R3, dans laquelle R3 représente un hydrogène, un groupe hydroxy, un groupe alcoxy en C₁-C₄, un groupe amino, un groupe alkylamino en C₁-C₄, un groupe alkyle en C₁-C₆ ou un groupe aryle ;
W représente un groupe cyano ou une fonction carbonyle (CO)-R4, dans laquelle R4 représente un hydrogène, un groupe hydroxy, un groupe alcoxy en C₁-C₄, un groupe amino, un groupe alkylamino en C₁₋C₄, un groupe alkyle en C₁-C₆ ou un groupe aryle ; en variante, V et W peuvent également former conjointement un système de noyau aliphatique ou aromatique, isocyclique ou hétérocyclique ; et
Kat⁺ correspond à un cation de métal alcalin, à un cation de métal alcalino-terreux, à un groupe ammonium quaternaire, à un groupe phosphonium quaternaire ou à un groupe sulfonium ;
à condition que W ne représente pas un groupe CO-phényle, un groupe CO-(4-bromophényle), un groupe CO-(4-chlorophényle), un groupe CO-(4-méthylphényle) ou un groupe CO-(4-méthoxyphényle), lorsque R1 représente un hydrogène, V représente un hydrogène ou un radical pyridyle non substitué, R2 représente un groupe nitro, X représente un oxygène et Y1 et Y2 représentent un atome d'azote.

3. Composé selon la revendication 1 ou 2, **caractérisé en ce que** le dérivé de 7-nitro-2,1,3-benzoxadiazole de formule (I) est choisi parmi le sel sodique de 4-(dicyanométhyl)-7-nitro-2,1,3-benzoxadiazole, le sel sodique de 4-(1-cyano-2-éthoxy-2-oxoéthyl)-7-nitro-2,1,3-benzoxadiazole, le sel sodique de 4-(dicyanométhyl)-7-nitro-2,1,3-benzoxadiazol-N-oxyde, le sel sodique de 4-(dihydro-2,4,6(1H,5H)-pyrimidinetrion-5-yl)-7-nitro-2,1,3-benzoxadiazole, le sel sodique de 4-(1-cyano-3,3-diméthyl-2-oxobutyl)-7-nitro-2,1,3-benzoxadiazole, le sel sodique de 4-(bis(méthoxycarbonyl)-7-nitro-2,1,3-benzoxadiazole, le sel sodique de 4-(4,5-dihydro-3-méthyl-1-phényl-1H-pyrazol-5-on-4-yl)-7-nitro-2,1,3-benzoxadiazole, le sel sodique de 4-(cyano-(4-nitro-phényl)-méthyl)-7-nitro-2,1,3-benzoxadiazole, le sel sodique de 4-((aminocarbonyl)-cyano-méthyl)-7-nitro-2,1,3-benzoxadiazole-1-oxyde, le sel sodique de 4-(1-cyano-2-éthoxy-2-oxo-éthyl)-7-nitro-2,1,3-benzoxadiazole-1-oxyde, le sel sodique de 4-(1,3-cyclohexanedion-2-yl)-7-nitro-2,1,3-benxoxadiazole, le sel sodique de 4-(carboxycyanométhyl)-7-nitro-2,1,3-benzoxadiazole, le sel sodique de 4-(2-éthoxy-1-nitro-oxoéthyl)-7-nitro-2,1,3-benzoxadiazole, le sel sodique de 4-((aminocarbonyl)cyanométhyl)-7-nitro-2,1,3-benzoxadiazole, le sel sodique de 4-(dihydro-2-thioxo-4,6(1H,5H)-pyrimidinedion-5-yl)-7-nitro-2,1,3-benzoxadiazole-1-oxyde, le sel sodique de 4-(1,3-dioxo-indan-2-yl)-7-nitro-2,1,3-benzoxadiazole, le sel sodique de 4-(2-oxo-2,3-dihydro-1H-indol-3-yl)-7-nitro-2,1,3-benzoxadiazole, le sel sodique de 4-(4-oxo-2-thioxo-thiazolidin-5-yl)-7-nitro-2,1,3-benzoxadiazole, le sel sodique de 4-(dihydro-6-thioxo-2,4-(1H,5H)-pyrimidinedion-3-yl)-7-nitro-2,1,3-benzoxadiazole, le sel sodique de 4-(1-cyano-2-oxo-2-phényléthyl)-2,1,3-benzoxadiazole et le sel sodique de 4-(cyano-(2-nitrophényl)-méthyl)-7-nitro-2,1,3-benzoxadiazole.

4. Composé selon la revendication 1 ou 2, **caractérisé en ce que** le dérivé de 7-nitro-2,1,3-benzthiadiazole de formule (I) est choisi parmi le sel sodique de 4-(dicyanométhyl)-7-nitro-2,1,3-benzthiadiazole, le sel sodique de 4-(1-cyano-2-éthoxy-2-oxoéthyl)-7-nitro-2,1,3-benzthiadiazole, le sel sodique de 4-(cyano-(4-nitrophényl)-méthyl)-7-nitro-2,1,3-benzthiadiazole, le sel sodique de 4-(dicyanométhyl)-7-nitro-2,1,3-benzthiadiazole-N-oxyde, le sel sodique de 4-(dihydro-2,4,6(1H,5H)-pyrrimidinetrion-5-yl)-7-nitro-2,1,3- benzthiadiazole, le sel sodique de 4-(1-cyano-3,3-diméthyl-2-oxobutyl)-7-nitro-2,1,3-benzthiadiazole, le sel sodique de 4-(bis(méthoxycarbonyl)-7-nitro-2,1,3-benzthiadiazole, le sel sodique de 4-(4,5-dihydro-3-méthyl-1-phényl-1H-pyrazol-5-on-4-yl)-7-nitro-2,1,3- benzthiadiazole, le sel sodique de 4-(1,3-cyclohexanedion-2-yl)-7-nitro-2,1,3-benzthiadiazole, le sel sodique de 4-(carboxycyanométhyl)-7-nitro-2,1,3-benzthiadiazole, le sel sodique de 4-(2-éthoxy-1-nitro-2-oxoéthyle)-7-nitro-2,1,3-benzthiadiazole, le sel sodique de 4-((aminocarbonyl)cyanométhyl)-7-nitro-2,1,3-benzthiadiazole, le sel sodique de 4-(dihydro-2-thioxo-4,6(1H,5H)-pyrimidinedion-5-yl)-7-nitro-2,1,3- benzthiadiazole-1-oxyde, le sel sodique de 4-(1,3-dioxo-indan-2-yl)-7-nitro-2,1,3-benzthiadiazole, le sel sodique de 4-(2-oxo-2,3-dihydro-1H-indol-3-yl)-7-nitro-2,1,3-benzthiadiazole, le sel sodique de 4-(4-oxo-2-thioxo-thiazolidin-5-yl)-7-nitro-2,1,3-benzthiadiazole, le sel sodique de 4-(dihydro-6-thioxo-2,4-(1H,5H)-pyrimidinedion-3-yl)-7-nitro-2,1,3- benzthiadiazole, le sel sodique de 4-(1-cyano-2-oxo-2-phényléthyl)-2,1,3- benzthiadiazole et le sel sodique de 4-(cyano-(2-nitrophényl)-méthyl)-7-nitro-2,1,3- benzthiadiazole.

5. Composition pour la teinture de fibres de kératine, **caractérisée en ce qu'**elle contient au moins un dérivé de 7-nitro-2,1,3-benzthiadiazole et/ou un dérivé de 7-nitro-2,1,3-benzthiadiazole de formule générale (I) selon l'une quelconque des revendications 1 à 4.

6. Compositions selon la revendication 5, **caractérisée en ce qu'**elle contient le composé de formule (I) en une quantité de 0,01 à 10% en poids.

7. Composition selon la revendication 5 ou 6, **caractérisée en ce qu'**elle contient, en plus du composé de formule (I), au moins un colorant direct supplémentaire parmi le groupe constitué de colorants anioniques, cationiques, non ioniques ou amphotères, de colorants nitro, de colorants azoïques, de colorants anthraquinones et de colorants de dispersion.

8. Composition selon l'une quelconque des revendications 5 à 7, **caractérisée en ce qu'**elle contient au moins un polymère habituel pour des compositions cosmétiques parmi le groupe constitué de polymères naturels ou synthétiques et de polymères modifiés d'origine naturelle et se présente sous la forme d'un fixateur de nuance ou d'un fixateur de couleur.

9. Composition selon l'une quelconque des revendications 5 à 8, **caractérisée en ce qu'**il s'agit d'une composition de teinture capillaire.

10. Utilisation de dérivés de 7-nitro-2,1,3-benzoxadiazole et/ou de dérivés de 7-nitro-2,1,3-benzthiadiazole de formule (I) selon l'une quelconque des revendications 1 à 4 en tant que colorant dans des compositions de teinture pour des fibres de kératine.
